# EUROPÄISCHE PATENTSCHRIFT

(11) **EP 0 713 685 B1**
(45) Veröffentlichungstag und Bekanntmachung des Hinweises auf die Patenterteilung: **28.06.2000**
(21) Anmeldenummer: 95117987.8
(22) Anmeldetag: 15.11.1995
(51) Int. Cl.: A61B 17/80

(54) **Gitterwerk für orthopädisch-traumatologische Chirurgie**
Mesh for orthopaedic traumatological surgery
Treillis pour chirurgie traumatologique orthopédique

(30) Priorität: 22.11.1994 IT MI942365
(43) Veröffentlichungstag der Anmeldung: 29.05.1996
(73) Patentinhaber: Martinotti, Lucio, 20124 Milano (IT)
(72) Erfinder: Martinotti, Lucio, 20124 Milano (IT)
(74) Vertreter: Jaumann, Paolo

(56) Entgegenhaltungen:
- CH-A- 611 147
- DE-U- 9 010 051
- DE-U- 9 114 118
- DE-U- 9 115 341
- FR-A- 2 466 977

## Beschreibung

Die vorliegende Erfindung betrifft ein neuartiges Gitterwerk für orthopädisch-traumatologische Chirurgie.

Aus der DE-U-9114118 ist ein Gitterwerk bekannt, das nur als spezielles Stützmittel für die Korrektur und/oder die Stabilisierung traumatisierter oder defizierter Wirbel der menschlichen Wirbelsäule verwendet werden kann.
Dieses Gitterwerk, geeignet insbesondere für die Anbringung im Halswirbel-bereich, ist aufgrund der notwendigen Versteifung im Längsmittenbereich und/oder der Schwächungen, nicht geeignet für eine Anwendung von fragmentarischen Knochenbüchen.
Aus der DE-U-9010051 ist eine Knochenplatte bekannt, die nur geeignet ist für den distalen Oberarmknochen und ist ebenfalls aufgrund der standard-mäßigen y-förmigen Biegung nicht geeignet für eine Anwendung bei zergliederten und zertrümmten Knochenbrüchen. Aufgabe der Erfindung ist die Schaffung eines Gitterwerkes für fragmentarische Knochenbüche, das dem Chirurg ermöglicht, die jeweils notwendinge Schrauben und/oder Nagelgröße mit mehr Freiheit zu wählen.

Das erfindungsgemässe Gitterwerk (aus Titan, Stahl oder verschiedenen Legierungen) besitzt drei voneinander an Durchmesser verschiedene, wabenförmig angeordnete Löcher. Diese Löcher haben einen Durchmesser im Bereich von 2,0 bis 6,5 mm und das Gitter= werk besitzt eine Wandstärke im Bereich von 0,5 bis 2 mm. Die drei Löcher mit unterschiedlichem Durchmesser ermöglichen die Verwendung von Schrauben mit unterschiedlichen Abmessungen. Dies ist besonders bei mehrfach zergliederten und zertrümmerten Knochenbrüchen von Vorteil.

Je nach Art des Knochenbruches bietet sich ein mehr oder weniger starkes Gitterwerk zur Verwendung an. Ein weiterer Vorteil der vorliegenden Erfindung besteht darin, dass das zweckentsprechend vorgestaltete Gitterwerk in Verbindung mit bekannten Platten verwendbar ist, wenn nur Platte und Gitterwerk aus gleichem Material bestehen.

Da auch Löcher mit kleinem Durchmesser vorgesehensind wird eine vor= läufige Befestigung mit Hilfe von leicht wiederentfernbaren Nägeln bzw. Stiften ermöglicht. Dies gestattet dem Chirurg mit mehr Freiheit und grösserer Präzision zu operieren.

In den beiliegenden Zeichnungen zeigen:

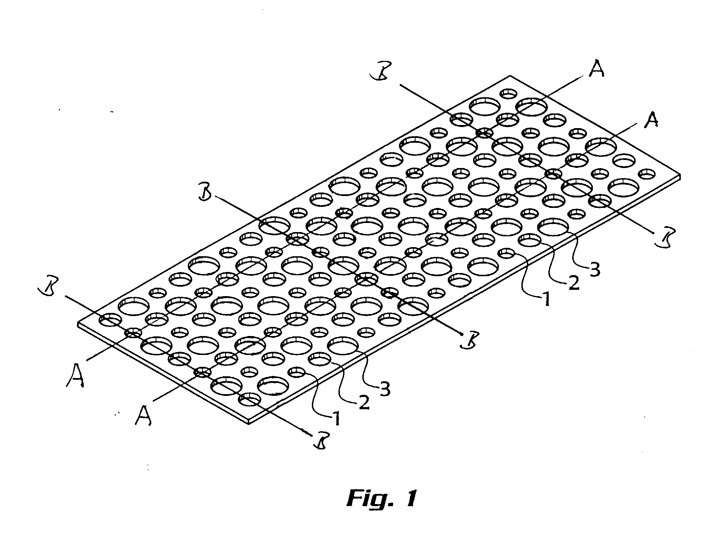

- Fig. 1 ein erfindungsgemässes Gitterwerk mit den drei Löchern (1, 2, 3) mit untereinander unterschiedlichem Durchmesser,
- Fig. 2 bis 11 verschiedenartige Anwendungen des Gitterwerkes bei Knochenbrüchen.

In Figur 9 ist beispielsweise eine kombinierte Verwendung des Gitter= werkes zusammen mit einer herkömmlichen Platte (6) dargestellt.

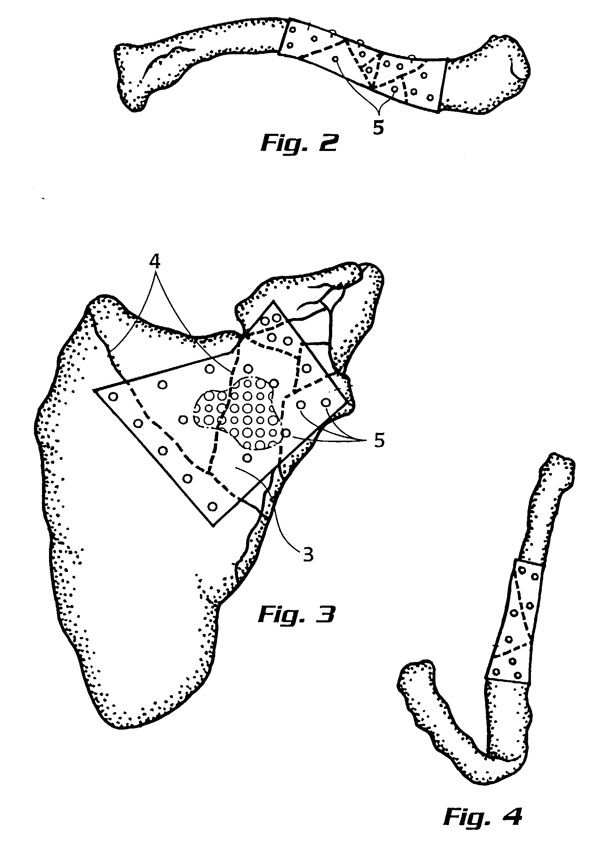

In den Figuren 2 bis 11 sind das Gitterwerk mit 3, die Knochenbrüche mit 4 und die Befestigungsschrauben mit 5 bezeichnet.

Die aufgezeigte Verwendung des erfindungsgemässen Gitterwerkes stellt in der orthopädisch-traumatologischen Chirurgie eine absolute Neuheit mit unzweifelhaften Vorteilen für die heilkräftige Behandlung von Knochenbrüchen dar.

### Figurenbeschreibung

Es zeigen:
- Fig. 2: Zerstückelte Knochenbrüche bzw. Pseudo-Arthrosis am Schlüssbein
- Fig. 3: Zerstückelte Knochenbrüche an der Knochenpfanne und am Knochenkörper des Schulterblattes

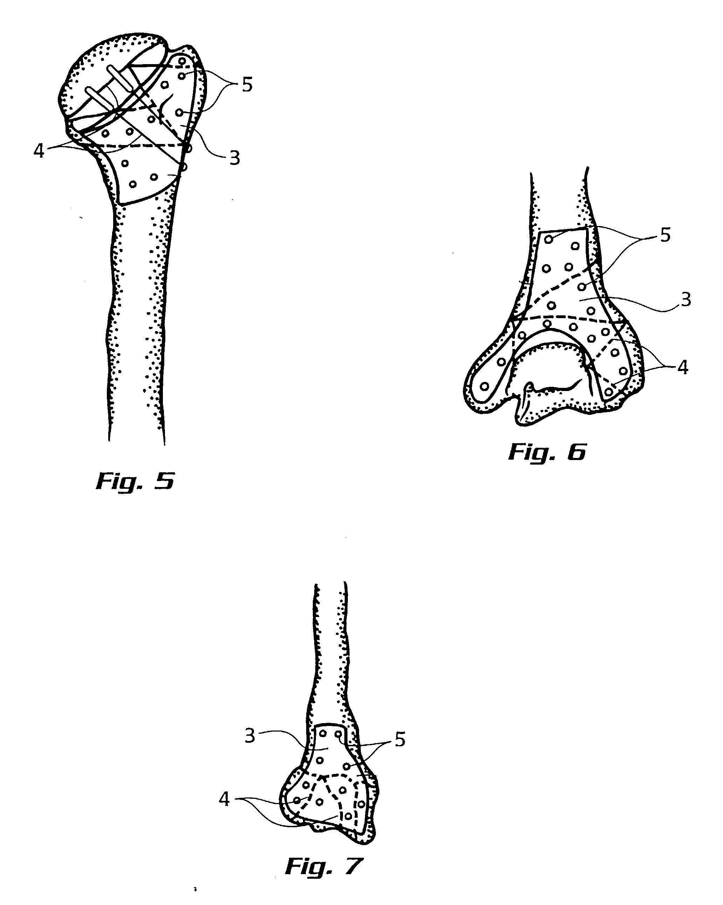

- Fig. 4: Zerstückelte Knochenbrüche an Rippen
- Fig. 5: Mehrfach fragmentarische Knochenbrüche am Knochentopf und am Knochenhals des Oberarmbeins
- Fig. 6: Zerstückelte Knochenbrüche am Oberarmblatt
- Fig. 7: Mehrfach fragmentarische Knochenbrüche des distalen Knochenansatzes des Speichenbeins

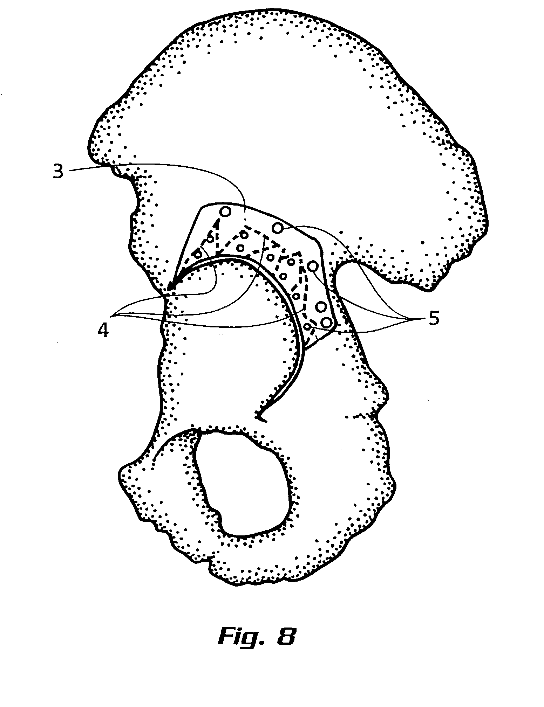

- Fig. 8: Mehrfach fragmentarischer, zerstückelter Knochenbruch an der Hüftgelenkpfanne

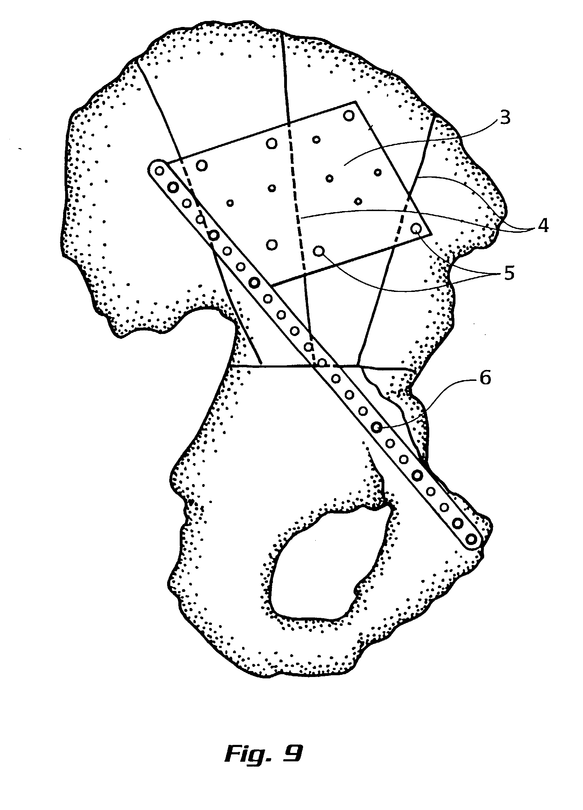

- Fig. 9: Mehrfach fragmentarischer Knochenbruch am Halbbecken, Hüftgelenkpfannendurchbrüche ggf.in Verbindung mit Platten (beckeneinwärts)

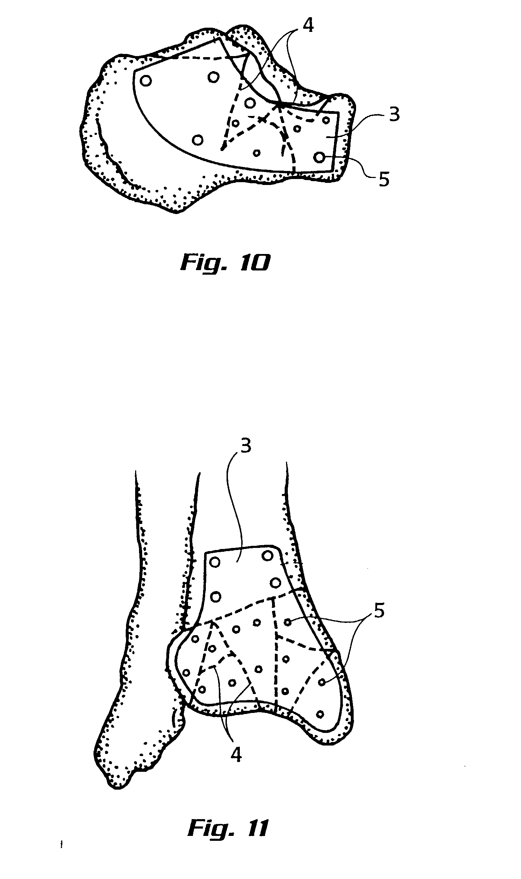

- Fig. 10: Mehrfach fragmentarische Knochenbrüche an der Ferse
- Fig. 11: Zerstückelte Knochenbrüche am Schienbein

## Patentansprüche

1. Gitterwerk für fragmentarische Knochenbrüche, geeignet für die Anwendung in der orthopädisch-traumatologischen Chirurgie mit drei Arten von Löchern (1, 2, 3) mit untereinander verschiedenem Durchmesser im Bereich von mindestens 2,0 mm bis höchstens 6,5 mm, wobei das Gitterwerk eine Stärke im Bereich von 0,5 bis 2 mm besitzt, und wobei in Längsrichtung (A) ebenso wie in Querrichtung (B) Gruppen von Löchern (1,2,3) mit kleinem, mittlerem und großem Durchmesser aufeinander folgend angeordnet sind.

2. Gitterwerk nach Anspruch 1, dadurch gekennzeichnet, dass das Gitterwerk in Verbindung mit Platten (6) verwendbar und vor Gebrauch vorgestaltbar ist.

3. Gitterwerk nach Anspruch 1 und 2, dadurch gekennzeichnet, dass in den Löchern mit kleinerem Durchmesser (1) leicht wiederentfernbare Nägel bzw. Stifte zur vorläufigen Befestigung des Gitterwerkes eingesetzt sind.

## Claims

1. Mesh for comminuted, fractured bone segments, used for orthopaedic traumatological surgery, having three groups of apertures (1, 2, 3) with different diameters ranging from 2,0 mm to 6,5 mm and having a thickness of 0,5 mm to 2 mm, wherein said groups of apertures with small, medium and large diameter (1, 2, 3) are arranged in columns (A) and trasversely-oriented rows (B) in repeating manner.

2. Mesh according to claim 1, characterized in that the mesh can be used in conjunction with plates (6) and can be formed before use.

3. Mesh according to claims 1 and 2, characterized in that in the apertures with small diameter (1) are introduced nails or pins, easily removable, for temporary fastening of the mesh.

## Revendications

1. Treillis pour fractures fragmentaires apte à l'application en chirurgie traumatologique orthopédique, avec trois types de trous (1, 2, 3) de diamètre différent l'un de l'autre compris entre au moins 2,0 mm et au plus 6,5 mm, le treillis présentant une épaisseur comprise entre 0,5 et 2 mm et en sens longitudinal (A) comme en sens transversal (B) étant disposés successivement des groupes de trous (1, 2, 3) de petit, moyen et grand diamètre.

2. Treillis selon la revendication 1, caractérisé en ce que le treillis est utilisable en liaison avec des plaques (6) et préfigurable avant usage.

3. Treillis selon les revendications 1 et 2, caractérisé en ce que des clous, resp. pointes facilement démontables sont placés dans les trous de plus petit diamètre (1) pour permettre une fixation provisoire du treillis.
